# EUROPEAN PATENT APPLICATION

(11) **EP 4 388 875 A1**
(43) Date of publication of application: **26.06.2024**
(21) Application number: 22857195.6
(22) Date of filing: 05.01.2022
(51) Int. Cl.: A01N 43/16, A01G 13/00, C12N 9/10, C12N 15/54, C12N 15/70, A01P 13/00

(54) **EFFECT OF FLAVONOID GLYCOSIDE IN RESISTANCE OF RICE TO WEEDS**

(30) Priority: 17.08.2021 CN 202110942015
(71) Applicant: Institute of Botany of The Chinese Academy of Sciences, Beijing 100093 (CN)
(72) Inventor: QI, Xiaoquan, Beijing 100093 (CN); MA, Aimin, Beijing 100093 (CN); SONG, Bo, Beijing 100093 (CN); DAI, Jungui, Beijing 100093 (CN); SONG, Zhijun, Beijing 100093 (CN)
(74) Representative: Isarpatent
(86) International application number: PCT/CN2022/070229
(87) International publication number: WO 2023/019871

(57) **Abstract**

Disclosed in the present invention is an effect of flavonoid glycoside in the resistance of rice to weeds. Provided in the present invention is the use of isoorientin in any one of the following: inhibiting the growth of weeds; regulating the allelopathy of plants; and regulating the resistance of plants to weeds. The results of the present invention show that isoorientin can regulate the allelopathy of plants and can be used for inhibiting the growth of weeds. The present invention is of great significance for developing an environment-friendly green pesticide, and provides theoretical guidance for controlling weeds in rice fields.

## Description

### TECHNICAL FIELD

The present invention relates to the technical field of biotechnology, and specifically to the use of a flavonoid glycoside in resistance of weeds in rice.

### BACKGROUND

Weed growth in rice fields seriously jeopardizes yield of rice and its quality (Amb and Ahluwalia, 2016; Chung et al., 2018). At present, the application of herbicides is the main way to control weeds, however, there are many hazards associated with the massive application of herbicides, such as environmental pollutions (Annett et al., 2014), endangering human health and increasing the number of weeds with resistance (Jabran et al., 2015). Therefore, more and more attention has been paid to exploring an environment-friendly weed control technology, especially the allelopathy-based weed control strategy (Cheng and Cheng, 2015). Interspecific phytoallelopathy refers to the phenomenon of mutualism between organisms, that is, a certain plant (or microorganism) releases one or more chemicals (allelochemicals) into the environment, thus directly or indirectly affecting the growth of another plant (or microorganism) (Rice, 1984).

At present, allelochemicals found in rice can be mainly categorized into indoles, phenolic acids, terpenoids, flavonoids, fatty acids, alkaloids and the likes (Khanh et al., 2007; Kato-Noguchi et al., 2011; Chung et al., 2018), in which the main flavonoid with allelopathy is tricin (5,7,4'-trihydroxy-3',5'-dimethoxyflavone), whereas it is not well understood whether other flavonoids have allelopathy, and the genetic and weed-suppressing mechanisms of rice allelopathy.

### SUMMARY OF THE INVENTION

The present invention seeks protection of a flavonoid glycoside in resistance of rice to weeds.

In a first aspect, the present invention seeks protection of use of isoorientin in any of the following:
P1, inhibiting the growth of weeds;
P2, regulating a allelopathy of plants; and
P3, regulating a resistance of plants to weeds.

The regulation of the allelopathy of plants may be increasing the content of isoorientin in the plant, so that the allelopathy of the plant is enhanced.

The regulation of the resistance of plants to weeds may be increasing the content of isoorientin in the plants, so that the resistance of the plants to weeds is enhanced.

In a second aspect, the present invention seeks protection of a method for inhibiting the growth of weeds.

The method for inhibiting the growth of weeds for which protection is sought by the present invention may include the following steps: applying isoorientin to weeds externally.

Wherein the working concentration of isoorientin may be more than 500 mg/L.

In a specific embodiment of the present invention, the working concentration of isoorientin is 500 mg/L.

In a specific embodiment of the present invention, the inhibition of the growth of weeds is specifically the inhibition of the growth of root or radicle of weeds.

In a third aspect, the present invention seeks protection of any of the following methods:
Method I: A method for breeding a plant variety with enhanced allelopathy includes the following steps: increasing the content of isoorientin in the recipient plant to obtain a plant variety with enhanced allelopathy.
Method II: A method for breeding a plant variety with weakened allelopathy includes the following steps: reducing the content of isoorientin in the recipient plant to obtain a plant variety with weakened allelopathy.
Method III: A method for breeding a plant variety with enhanced weed resistance, which includes the following steps: increasing the content of isoorientin in the recipient plant to obtain a plant variety with enhanced weed resistance.
Method IV: A method for breeding a plant variety with weakened weed resistance includes the following steps: reducing the content of isoorientin in the recipient plant to obtain a plant variety with weakened weed resistance.

In the method, the content of isoorientin in the recipient plant may be increased by:
(A1) reducing the activity and/or expression of protein which is capable of catalyzing the transformation of isoorientin into other substances in the recipient plant; and/or
(A2) increasing the activity and/or expression of protein which is capable of catalyzing the transformation of other substances into isoorientin in the recipient plant.

In the method, the content of isoorientin in the recipient plant may be reduced by:
(B1) increasing the activity and/or expression of protein which is capable of catalyzing the transformation of isoorientin into other substances in the recipient plant; and/or
(B2) reducing the activity and/or expression of protein which is capable of catalyzing the transformation of other substances into isoorientin in the recipient plant.

Further, the (A1) may be implemented by the following (a1); and the (A2) may be implemented by the following (a2):
(a1) inhibiting the expression of a gene encoding a protein which is capable of catalyzing the transformation of isoorientin into other substances in the recipient plant; and
(a2) introducing a gene encoding a protein which is capable of catalyzing the transformation of other substances into isoorientin in the recipient plant.

Further, the (B1) may be implemented by the following (b1); and the (B2) may be implemented by the following (b2):
(b1) introducing a gene encoding a protein which is capable of catalyzing the transformation of isoorientin into other substances in the recipient plant; and
(b2) inhibiting the expression of a gene encoding a protein which is capable of catalyzing the transformation of other substances into isoorientin in the recipient plant.

Among them, the other substances are substances that is not capable of improving the allelopathy of the recipient plant, or substances that improve the allelopathy of the recipient plants less than isoorientin. Alternatively, the other substances are substances that is not capable of improving the weed resistance to the recipient plants, or substances that improve the weed resistance to the recipient plants less than isoorientin.

In a specific embodiment of the present invention, in the (A1), the (a1), the (B1) and the (b1), the other substance is specifically isoorientin-2"-O-glucopyranoside.

In the above aspects, the plant may be rice; and the weed may be *Echinochloa crus-galli.*

In a fourth aspect, the present invention seeks protection of the use of Os02g0589400 protein or a relevant biomaterial thereof in any of the following:
Q1, catalyzing the glycosylation reaction of isoorientin;
Q2, preparing isoorientin-2"-O-glucopyranoside; and
Q3, regulating the content of isoorientin in plants.

The relevant biomaterial is a nucleic acid molecule capable of expressing the Os02g0589400 protein or an expression cassette, a recombinant vector, a recombinant bacterium or a transgenic cell line containing the nucleic acid molecule.

The Os02g0589400 protein may be any one of that following protein:
(D1) a protein with the amino acid sequence of SEQ ID No.1;
(D2) a protein having one or more amino acid residues substituted and/or deleted and/or added in the amino acid sequence shown in SEQ ID No.1 and having the same function;
(D3) a protein having 99% or more, 95% or more, 90% or more, 85% or more or 80% or more homology with the amino acid sequence defined in any one of (D1) to (D2) and having the same function; and
(D4) a fusion protein obtained by attaching a tag to the N-terminal and/or C-terminal of the protein defined in any one of (D1) to (D3).

Of the above-mentioned proteins, the tag refers to a polypeptide or protein that is fused and expressed with the target protein by using DNA *in vitro* recombination technology, so as to facilitate the expression, detection, tracing and/or purification of the target protein. The tag may be a Flag tag, a His tag, an MBP tag, an HA tag, an myc tag, a GST tag and/or a SUMO tag and the likes.

Of the above-mentioned proteins, the identity refers to the identity to an amino acid sequence. The identity to the amino acid sequence may be determined using the homology search site on the international internet, such as BLAST webpage on the NCBI homepage website. For example, the value of identity (%) may be obtained by retrieving the identity of a pair of amino acid sequences for calculation in the advanced BLAST2.1, using blast program, setting the Expect value to 10, all Filters to OFF, using BLOSUM62 as Matrix, setting Gap existence cost, Per residual gap cost, and Lambda ratio to 11, 1, and 0.85(default values), respectively.

Of the above-mentioned proteins, the identity of 99% or more may be an identity of at least 99.1%, 99.2%, 99.3%, 99.4%, 99.5%, 99.6%, 99.7%, 99.8%, 99.9% or 100%. The identity of 95% or more may be an identity of at least 96%, 97% and 98%. The identity of 90% or more may be an identity of at least 91%, 92%, 93% and 94%. The identity of 85% or more may be an identity of at least 86%, 87%, 88% and 89%. The identity of 80% or more may be an identity of at least 81%, 82%, 83% and 84%.

The nucleic acid molecule that is capable of expressing the Os02g0589400 protein may be any of the following DNA molecules:
(E1) a DNA molecule shown in SEQ ID NO.2;
(E2) a DNA molecule that hybridizes to the DNA molecule defined in (E1) under stringent conditions and encodes the Os02g0589400 protein;
(E3) a DNA molecule having 99% or more, 95% or more, 90% or more, 85% or more or 80% or more homology with the DNA sequence defined in (E1) or (E2) and encodes the Os02g0589400 protein.

Of the above-mentioned nucleic acid molecules, the stringent conditions may be as follows: hybridizing in a mixed solution of 7% sodium dodecyl sulfate (SDS), 0.5 M Na₃PO₄, and 1 mM EDTA at 50°C, followed by rinsing in 2 × SSC and 0.1% SDS at 50°C; alternatively, hybridizing in a mixed solution of 7% SDS, 0.5 M Na₃PO₄, and 1 mM EDTA at 50°C, followed by rinsing in 1 × SSC and 0.1% SDS at 50°C; alternatively, hybridizing in a mixed solution of 7% SDS, 0.5 M Na₃PO₄, and 1 mM EDTA at 50°C, followed by rinsing in 0.5 × SSC and 0.1% SDS at 50°C; alternatively, hybridizing in a mixed solution of 7% SDS, 0.5 M Na₃PO₄, and 1 mM EDTA at 50°C, followed by rinsing in 0.1 × SSC and 0.1% SDS at 50°C; alternatively, hybridizing in a mixed solution of 7% SDS, 0.5 M Na₃PO₄, and 1 mM EDTA at 50°C, followed by rinsing in 0.1 × SSC and 0.1% SDS at 65°C; alternatively, hybridizing in a solution of 6 × SSC and 0.5% SDS at 65°C, followed by washing the membrane once each in 2 × SSC and 0.1% SDS and 1 × SSC and 0.1% SDS.

Of the above-mentioned nucleic acid molecules, the homology refers to the identity to the nucleotide sequence. The identity to the nucleotide sequence may be determined using the homology search site on the international internet, such as BLAST webpage on the NCBI homepage website. For example, the value of identity (%) may be obtained by retrieving the identity of a pair of nucleotide sequences for calculation in the advanced BLAST2.1, using blast program, setting the Expect value to 10, all Filters to OFF, using BLOSUM62 as Matrix, and setting Gap existence cost, Per residual gap cost, and Lambda ratio to 11, 1, and 0.85(default values), respectively.

Of the above-mentioned nucleic acid molecules, the homology of 95% or more may be an identity of at least 96%, 97%, and 98%. The homology of 90% or more may be an identity of at least 91%, 92%, 93%, and 94%. The homology of 85% or more may be an identity of at least 86%, 87%, 88%, and 89%. The homology of 80% or more may be an identity of at least 81%, 82%, 83%, and 84%.

The recombinant expression vector may be constructed by existing plant expression vectors. The plant expression vectors include binary Agrobacterium tumefaciens vectors and vectors that can be used for biolistics, such as pCAMBIA-1300-221, pGreen0029, pCAMBIA3301, pCAMBIA1300, pBI121, pBin19, pCAMBIA2301, pCAMBIA1301-UbiN or other derived plant expression vectors. The plant expression vector may further contain the 3' end untranslated region of the exogenous gene, that is, it contains polyadenylation signals and any other DNA fragments involved in mRNA processing or gene expression. The polyadenylation signal may direct the incorporation of polyadenylate into the 3' end of the mRNA precursor. When recombinant expression vectors are constructed using the gene, any one of the enhanced, constitutive, tissue-specific or inducible promoters, such as the cauliflower mosaic virus (CAMV) 35S promoter, the ubiquitin gene Ubiquitin promoter (pUbi), the stress-inducible promoter rd29A, etc., which may be used alone or in combination with other plant promoters, may be added prior to the nucleotide at the transcriptional start site; in addition, when recombinant expression vectors are constructed using the gene of the present inventio, enhancers may also be used, including translation enhancers or transcription enhancers. These enhancer regions may be ATG start codon or adjacent region start codon, and the likes, but they must be the same as the reading frame of the coding sequence to ensure the correct translation of the whole sequence. The source of the translation control signal and the start codon is extensive, which may be natural or synthetic. The translation initiation region may be derived from a transcription initiation region or a structural gene. In order to facilitate the identification and screening of transgenic plant cells or plants, the recombinant expression vector may be processed, such as adding genes that can be expressed in plants and encode enzymes or luminescent compounds that produce color changes, antibiotic markers with resistance or chemical reagent marker genes. It is also possible to directly screen transformed plants by adversity without adding any selective marker genes.

In a fifth aspect, the present invention also seeks protection of the plant varieties breeded by the method described in the preceding third aspect.

The plant varieties include, but are not limited to, whole plants, seeds, reproductive tissues or organs, etc.

The plant variety may be any one of the following depending on different cultivation methods: plant variety with enhanced allelopathy, plant variety with weakened allelopathy, plant variety with enhanced weed resistance and plant variety with weakened weed resistance.

In a sixth aspect, the present invention also seeks protection of a compound for inhibiting the growth of weeds.

The compound for inhibiting the growth of weeds for which protection is sought by the present invention is isoorientin.

Wherein the weed may be *Echinochloa crus-galli.*

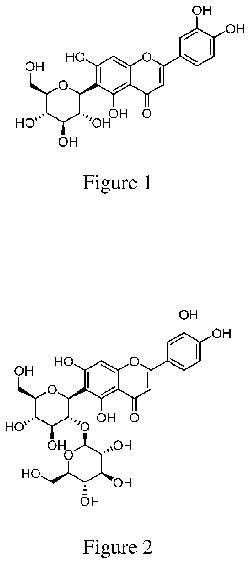

In the above aspects, the structural formula of isoorientin is shown in Figure 1. And the structural formula of isoorientin-2"-O-glucopyranoside is shown in Figure 2.

### BRIEF DESCRIPTION OF DRAWINGS

Figure 1 shows the structural formula of isoorientin.
Figure 2 shows the structural formula of isoorientin-2"-O-glucopyranoside.
Figure 3 shows the purification of Os02g0589400 protein. Wherein the arrow indicates the size of the recombinant protein after protein purification.
Figure 4 is a chromatogram diagram showing the product of Os02g0589400 from enzyme activity assay *in vitro.*
Figure 5 shows the comparison of the herbicidal effects between compounds isoorientin and isoorientin-2"-O-glucopyranoside. A shows the effect of isoorientin and isoorientin-2"-O-glucopyranoside on the growth of radicle of *Echinochloa crus-galli*; B shows the effect of isoorientin and isoorientin-2"-O-glucopyranoside on the growth of plumule *of Echinochloa crus-galli.*

### DETAILED DESCRIPTION

The following examples facilitate a better understanding of the present invention, but are not intended to limit the present invention. The methods used in the experiments of the following examples are all conventional methods unless otherwise specified. The materials used in the following examples are all commercially available from the conventional biochemical reagent stores unless otherwise specified. All the quantitative experiments in the following examples were performed in triple, and the results were averaged.

### Example 1. Purification of the Compounds

(1) Fresh leaves of Indica rice varieties with high target compound content about 55 days after sowing were collected, and ground into powder after drying by freeze dryer. 8 times of 75% ethanol (m/v) was added, and extracted ultrasonically for 3 times, 20min each time.
(2) The extract was filtered, concentrated with a rotary evaporator until it has no alcohol smell, into which petroleum ether of equal volume was added to be extracted for 3 times, then the extract from the petroleum ether phase was collected and an equal volume of dichloromethane was added into the remaining aqueous phase to extract for 3 times, then the extract from dichloromethane phase was collected, an equal volume of n-butanol was added into the remaining aqueous phase to extract for 3 times, the extract from n-butanol phase was collected.
(3) 1 mL of the extract from each phase in (2) was taken, respectively, dissolved with methanol after being dried, centrifuged, and the supernatant was aspirated into an Agilent sampling bottle, and the compounds in each phase were detected by UPLC-QTOF/MS. Chromatographic column: RRHD SB-C18 (Agilent), particle size of packing: 1.8 µm, specification: 2.1 mm (inner diameter) ×100 mm (length). Column temperature: 40°C, injection volume: 5 µL. Mobile phase A: H₂O (containing 0.1% formic acid, % represents volume percentage); phase B: acetonitrile. Elution gradient: 0-2 min: 5% B-10% B, 2-12 min: 10% B-25% B, 12-18 min: 25% B-70% B, 18-23 min: 70% B-90% B, 23-25 min: 90% B - 100% B, 25-30 min: 100% B, then running for 5 min. The flow rate was 0.3 mL/min, and the electrospray ionization source (ESI) was adopted as the mass spectrometry system with positive ion detection mode, the carrier gas was high-purity nitrogen, pressure: 40 psi, temperature: 325°C. After detection, the target compound mainly exists in n-butanol phase.
(4) The resulting n-butanol phase was concentrated by a rotary evaporator, and then it was subjected to crude fractionation using a medium-low pressure rapid preparative liquid chromatograph (BiotageIsolera^{™} Prime) by wet sampling. Silica gel column packing: YMC*GEL C18 spherical packing, packing particle size: 50 µm, pore size: 12 nm. Mobile phase: water (containing 0.1% formic acid, % represents volume percentage) and methanol. The proportion of mobile phase were as follows: 10%-90% of methanol in 10 columns, 90%-100% in 2 columns, wherein % represents the volume percentage. Flow rate: 50 mL/min.
(5) The content of the target compound in each fraction was detected by UPLC-QTOF/MS, and the detection method was the same as that in (3). Then the fractions containing the target compound were combined and concentrated by rotary evaporator.
(6) The concentrate was subjected to crude fractionation by ODS chromatographic column and eluted sequentially with 18:82, 24:76, 30:70, 35:65, 40:60, 50:50, 60:40, 70:30, 80:20, 100:00 (v/v) methanol:H₂O solution. 8 fractions were obtained, and then the content of the target compound in each fraction was detected by UPLC-QTOF/MS. After detection, the target compound was found in Fraction 2(Fr2) and Fraction 3(Fr3).
(7) Fr2 was weighed after concentration. Then 203 mg of FR2 was separated by reversed-phase semi-preparative high performance liquid chromatography. Mobile phase: acetonitrile-H₂O(15:85, v/v), flow rate: 2.5mL/min. A total of 13 fractions (Fr2.1-Fr2.13) were obtained. The target compound was detected in Fr2.10. After concentrating it, 43 mg of concentrate was obtained, and then it was further separated by reversed-phase semi-preparative high performance liquid chromatography. Mobile phase: methanol-H₂O (40:60, v/v), flow rate: 2.5mL/min, isoorientin-2"-O-glucopyranoside (13.0 mg, RT=18.3 min) was obtained.
(8) After concentration, 108 mg of concentrate was obtained from Fr3. Then it was subjected to crude fractionation by Sephadex LH-20 chromatographic column, and the eluent was methanol. A total of 8 fractions were obtained, of which fraction 2(Fr3.2) contained the target compound. After concentration, Fr 3.2(54 mg) was purified by reversed-phase semi-preparative high performance liquid chromatography. Mobile phase: acetonitrile-H₂O (20:80, v/v), flow rate: 2.5 mL/min, isoorientin) (16.9 mg, RT=16.3 min) was obtained.
(9) The obtained compound monomer was structurally characterized by NMR (Bruker AVIII 400 spectrometer). The structure of isoorientin is shown in Figure 1, and the specific NMR results are as follows: ¹H-NMR (400 MHz, Methanol -d4) *δ*: 6.53 (1H, s, H-3), 6.47 (1H, s, H-8), 7.35 (1H, s, H-2'), 6.89 (1H, brs H-5'), 7.35 (1H, s, H-6'), 4.89 (1H, d, *J* = 8.0 Hz, H-1"), 4.18 (1H, m, H-2"), 3.48 (1H, m, H-3"), 3.50 (1H, m, H-4"), 3.43 (1H, m, H-5"), 3.91 (1H, d, *J* = 12.4 Hz, H-6a"), 3.75 (1H, dd, *J* =12.4, 5.2 Hz, H-6b"),;¹³C-NMR (100 MHz, Methanol-d4) *δ*: 166.2 (C-2), 103.9 (C-3), 184.0 (C-4), 162.0 (C-5), 109.1 (C-6), 164.8 (C-7), 95.2 (C-8), 158.7 (C-9), 105.2 (C-10), 123.5 (C-1'), 114.1 (C-2'), 147.0 (C-3'), 151.0 (C-4'), 116.8 (C-5'), 120.3 (C-6'), 75.3 (C-1"), 72.6 (C-2"), 80.1 (C-3"), 71.8 (C-4"), 82.6 (C-5"), 62.9 (C-6").

The structure of isoorientin-2"-O-glucopyranoside is shown in Figure 2, and the specific NMR results are as follows: ¹H-NMR (400 MHz, DMSO-d6) *δ*: 13.65 (1H, s, 5-OH), 7.35 (1H, s, H-6'), 7.35 (1H, s, H-2'), 6.84 (1H, brs H-5'), 6.54 (1H, s, H-3), 6.40 (1H, s, H-8), 4.65 (1H, d, *J* = 8.0 Hz, H-1"), 4.52 (1H, m, H-2"), 3.41 (1H, m, H-3"), 3.18 (1H, m, H-4"), 3.18 (1H, m, H-5"), 3.19 (1H, m, H-6"), 4.22 (1H, d, *J =* 8.5 Hz, H-1‴), 2.85 (1H, m, H-2‴), 3.04 (1H, m, H-3‴), 3.04(1H, m, H-4‴), 3.04 (1H, m, H-5‴), 3.66 (1H, m, H-6‴);¹³C-NMR (100 MHz, DMSO-d6) *δ*: 163.3.2 (C-2), 102.3 (C-3), 181.6 (C-4), 160.5 (C-5), 108.2 (C-6), 161.5 (C-7), 93.2 (C-8), 156.9 (C-9), 104.7 (C-10), 121.1 (C-1'), 112.8 (C-2'), 146.0 (C-3'), 150.6 (C-4'), 116.11 (C-5'), 118.9 (C-6'), 71.6 (C-1"), 81.3 (C-2"), 78.6 (C-3"), 70.5 (C-4"), 81.6 (C-5"), 60.7 (C-6"), 105.6 (C-1‴), 74.6 (C-2‴), 76.6 (C-3‴), 69.8 (C-4‴), 76.6 (C-5‴), 61.7 (C-6‴).

### Example 2. The Application of Os02g0589400 Gene

### (1) Gene cloning

The gene *Os02g0589400* was cloned by using the genomic DNA from seedling stage leaves of *Oryza sativa* L. *ssp. japonica* cv. Zhonghua 11 (ZH11) as a template. The target gene was ligated into pEASY-T3 vector (TransGen Biotech, pEASY@-T3 Cloning Kit) by enzyme digestion and ligation. Then the resulting product was transformed into DH5α competent cells, and the positive clones were screened by blue-white selection. The obtained recombinant vector plasmid was named pEASY-T₃-*Os02g0589400.*

The structure of the recombinant vector pEASY-T₃*-Os02g0589400* is described as a recombinant plasmid in which the small fragment between the restriction sites *BamHI* and *Hind*III of pEASY-Ts vector is replaced by the DNA fragment shown in SEQ ID No.2. SEQ ID No.2 is the CDS sequence of *Os02g0589400* gene, which encodes the protein shown in SEQ ID No.1.

### (2) Prokaryotic expression of Os02g0589400

The recombinant vector pEASY-T₃*-Os02g0589400* was completely digested with restriction endonucleases *BamHI* and Hind*III,* and the expression vector pMAL-c2X (Huayueyang Biotechnology CO., LTD., VECT-570) was also digested. The digested products were subjected to gel electrophoresis, and the fragments containing *Os02g0589400* with a size of about 1.3 Kb and the pMAL-c2X vector fragment with a size of about 6.6 Kb were recovered and ligated. The recombinant plasmid obtained was named *pMAL-Os02g0589400* after sequencing verification. The structure of *pMAL-Os02g0589400* is described as a vector obtained by replacing the sequence between *BamHI* and Hind*III* of pMAL-c2X vector with *Os02g0589400* gene (SEQ ID No.2).

The obtained recombinant prokaryotic expression vector *pMAL-Os02g0589400* was transformed into competent cells of *Escherichia coli* NovaBlue (Huayueyang Biotechnology CO., LTD., WR4478), and a pMAL-c2X mock was used as control. Positive clones were screened by LB plate containing Ampicillin antibiotic, and identified by PCR amplification method. The primers were F1/R1, and the amplified fragment was 414 bp in size.

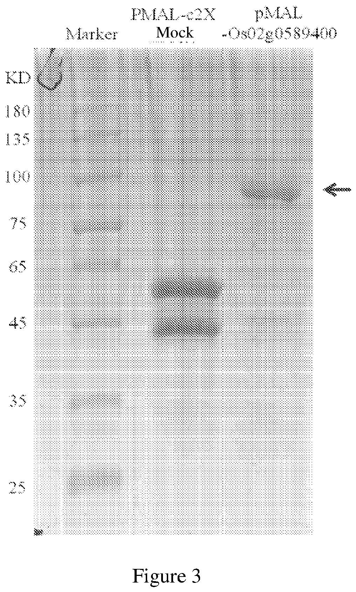

The positive clones were inoculated into 20 mL of LB liquid medium containing Ampicillin, and incubated at 37°C, 220 rpm for 12-14 hours. Then the bacterial solution was transferred to 400 mL of LB liquid medium (containing 100 µg/mL Ampicillin) at a ratio of 1:1000 (v/v), 37°C, 220 rpm, and incubated until OD₆₀₀ = 0.6-0.8. The bacterial solution was taken out and cooled on ice, then 160 µL of IPTG with a concentration of 500 mM was added so that the final concentration of IPTG was 0.2 mM. Bacteria were incubated at 16°C with 100 rpm shaking for 24 hours to induce protein expression. The bacteria were centrifuged at 4°C, 10,000 rpm for 10 min and collected. Then the obtained bacteria were resuspended with column buffer and frozen at -20°C overnight. The formula of column buffer (1L) was as follows: NaCl 11.7g; DTT 154 mg; 0.5 M EDTA 2 mL; 1 M Tris-HCl (pH = 7.4) 40 mL; The remainer is water. After the samples were melted on the next day, the cells were crushed by ultrasonic crusher, and then centrifuged at 10,000 rpm for 10 min. The target protein was purified by amylose column. The specific purification process was as follows:
a. The affinity column packing was activated with column buffer solution (see the formula above) (flow rate of 1 mL/min).
b. The crude protein supernatant obtained was slowly added into the activated amylose column, and the flow rate is adjusted to about 0.5 mL/min, so as to make the target protein and the packing of the affinity column fully combined.
c. After all the samples flow through the packing, column buffer was added and rinsed several times to remove unbound proteins.
d. 15 mL of column buffer containing maltose (1 L of column buffer contains 3.6 g of maltose) was added to elute the target protein, and repeat the step once.
e. The eluent was added into the ultrafiltration tube in several times, and centrifuged at 4°C, 5000 rpm for 15 min after each addition.
f. 1 mL of 100 mM Tris-HCl was added, centrifuged at 4°C, 5000 rpm for 15min, and the waste solution was poured off. The target protein solution was transferred into a 1.5 mL Eppendorf centrifuge tube.
g. 2 µL of the obtained target protein solution was aspirated, into which 1 mL of Bradford (Quick Start^{™} Bradford 1× Dye Reagent, BioRAD, CAS 67-56-1) was added, and the absorption peak of the protein to be tested was measured at 595 nm (OD₅₉₅) by using a spectrophotometer. Then the absorption value of the target protein was calculated into protein concentration, and the concentration of Os02g0589400 recombinant protein was 2.03 µg/µL.
h. The molecular weight of the recombinant protein Os02g0589400 was confirmed to be about 90 KD in size by SDS-PAGE electrophoresis with coomassie brilliant blue staining (Fig. 3).
   Primer F1: 5'-GGCGACACAGCTCTCATGTCCTCGT-3';
   Primer R1: 5'-CCCTCTCGCCCCTTCCAAGTAGCTC-3'.

### (3) In vitro enzymatic activity of Os02g0589400

The enzymatic activity reaction system was: 38 µL of Tris-HCl (pH 7.0, 50 mM, containing 10 mM DTT), 1µL of 10 mM glycosyl receptor (the glycosyl receptor of Os02g0589400 is isoorientin), 1 µL of 100 mM glycosyl donor (UDPG), and 10 µL of 2.03 µg/µL of recombinant protein of Os02g0589400 obtained in (2). React in water bath at 30°C for 1 hour, and then stop the reaction with equal volume of methanol. A sample containing protein obtained from *E. coli* into which a pMAL-c2X mock vector was transferred according to the above step (2) was taken as the negative control. Centrifuge at 12,000 rpm, 4°C for 10 min, 30 µL of the resulting supernatant was taken and measured by UPLC-QTOF/MS, and the measuring method was the same as that shown in Example 1.

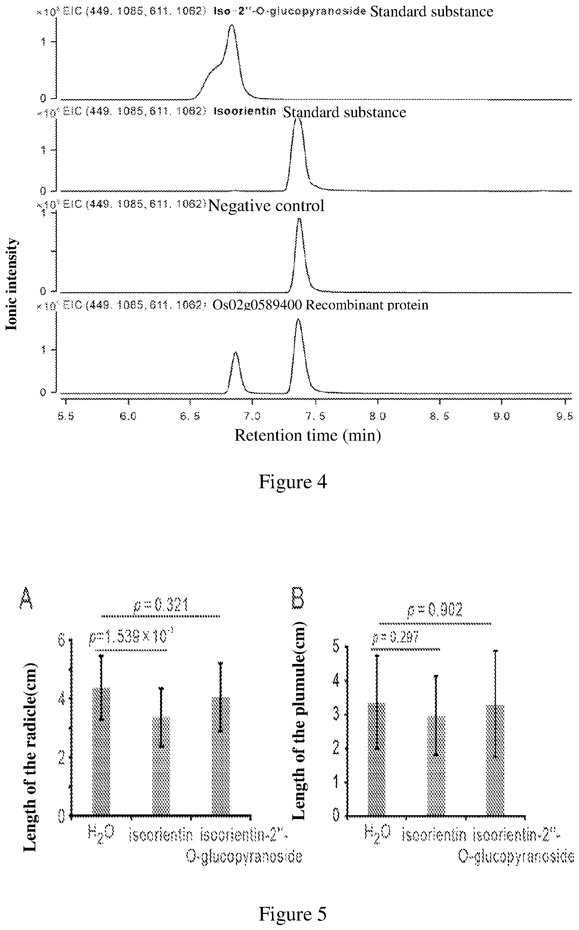

The results showed that compared with the mock control group, in the reaction system into which Os02g0589400 recombinant protein was added, Os02g0589400 was could catalyze isoorientin to produce isoorientin-2"-O-glucopyranoside, indicating that Os02g0589400 plays an important role in catalyzing isoorientin to produce isoorientin-2"-O-glucopyranoside (as shown in Fig. 4).

### Example 3. External Application of the Compound to Lettuce and Echinochloa crus-galli

### I. Procedures for external application of the compound to lettuce and Echinochloa crus-galli

Compounds to be tested: isoorientin and isoorientin-2"-O-glucopyranoside.
(1) The compounds to be tested were dried into powder with a centrifugal concentrator and weighed, a proper amount of sterile water was added to make its final concentration to be 10 mg/mL.
(2) 500 mL of 0.8% agar was prepared and sterilized in an autoclave.
(3) The sterilized agar was put on a super-clean bench and cooled to room temperature (note: do not allow the medium to solidify), and then 4.75 mL of medium was aspirated accurately into a sterilized 10 mL centrifuge tube. 250 µL solution of the compounds to be tested at a concentration of 10 mg/mL were added to make the final concentration of the compounds to be 500 mg/L. The medium containing the compound to be tested was quickly poured into a 10 cm×10 cm square dish, and the square dish was shaken quickly so that the medium was evenly spread in the square dish. The medium added with equal amount of sterile water was used as control.
(4) The seeds of *Echinochloa crus-galli* were washed with distilled water for several times, and then placed on sterile filter paper to dry.
(5) After the medium had solidified, the seeds of *Echinochloa crus-galli* were placed with sterile tweezers on the medium, and 26 seeds were placed in each petri dish (2 rows, with 13 seeds in each row). Each treatment had 3 biological repeats.
(6) The petri dishes were placed in an incubator at 28°C for 3 days in the dark.
(7) Phenotypic statistics: after 3 days, the length of radicle and embryo of each treated material were counted.

### II. Phenotypes of Echinochloa crus-galli and lettuce after external application of the compound

As shown in Fig. 5, isoorientin and isoorientin-2"-O-glucopyranoside were applied to *Echinochloa crus-galli* externally at a concentration of 500 mg/L, respectively. Compared with the control, the radicle length of *Echinochloa crus-galli* after isoorientin treatment was significantly shortened (*p* =1.539×10⁻³); however, there was no significant difference in the radicle length of *Echinochloa crus-galli* treated with isoorientin-2"-O-glucopyranoside compared with the control (p = 0.321). In addition, there was no significant difference in the embryo length of *Echinochloa crus-galli* after isoorientin and isoorientin-2"-O-glucopyranoside treatments, respectively, compared with the control *(p* = 0.297, *p* = 0.902). The results showed that the external application of the compound isoorientin mainly affected the growth of radicle of *Echinochloa crus-galli*, and compared with the its product, isoorientin-2"-O-glucopyranoside, isoorientin had a more significant inhibitory effect on the growth of radicle of *Echinochloa crus-galli.*

The present invention has been described in detail above. For those skilled in the art, the present invention may be implemented in a wide range with equivalent parameters, concentrations and under equivalent conditions without departing from the spirit and scope of the present invention and without unnecessary experiments. Although the present invention has given specific examples, it should be understood that the present invention may be further modified. In summary, the present application is intended to include any changes, uses or improvements to the present invention according to the principles of the present invention, including changes made by conventional techniques known in the art, which depart from the disclosed scope in this application. According to the scope of the following appended claims, some basic features can be applied.

### INDUSTRIAL APPLICATIONS

The present invention provides a flavonoid glycoside, isoorientin, that is associated with allelopathy. External application experiment of the compound showed that isoorientin had a significant inhibitory effect on the growth of radicle of *Echinochloa crus-galli.* The gene involved in the formation of isoorientin compound provided by the invention is *Os02g0589400. In vitro* enzyme activity assays showed that *Os02g0589400* could catalyze isoorientin to form a glycosylated product isoorientin-2"-O-glucopyranoside. Isoorientin has a more significant inhibitory effect on the growth of radicle of *Echinochloa crus-galli* compared with the product isoorientin-2"-O-glucopyranoside when applied externally at a concentration of 500 mg/L. It can be seen that isoorientin could regulate the allelopathy of plants and could be used to inhibit the growth of weeds. The present invention is of great significance for developing an environment-friendly green pesticide, and provides theoretical guidance for controlling weeds in rice fields.

## Claims

1. Use of isoorientin in any one of the following:
P1, inhibiting a growth of weeds;
P2, regulating a plant allelopathy ; and
P3, regulating a plant resistance to weeds.

2. The use according to claim 1, **characterized in that** in P2, the regulation of the allelopathy of plants is capable of increasing the content of isoorientin within the plant, thereby enhancing the plant allelopathic effect; and
in P3, the regulation of a plant resistance to weeds is capable of increasing the content of isoorientin in the plant,thereby enhancing the plant resistance to weeds.

3. The use according to claim 1 or 2, **characterized in that** the plant is rice; and the weed is *Echinochloa crus-galli.*

4. A method for inhibiting the growth of weeds, the method comprising the following step: applying isoorientin externally to the weeds.

5. The method according to claim 4, **characterized in that** an effective concentration of the isoorientin is more than 500 mg/L.

6. The method according to claim 5, **characterized in that** an effective concentration of the isoorientin is 500 mg/L.

7. The method according to any one of claims 4-6, **characterized in that** the inhibition of weed growth is the inhibition of root or radicle of weeds.

8. A method for breeding a plant variety with enhanced allelopathy, the method comprising the following step: increasing the content of isoorientin in a recipient plant to obtain a plant variety with enhanced allelopathy.

9. A method for breeding a plant variety with reduced allelopathy, the method comprising the following step: decreasing the content of isoorientin in a recipient plant to obtain a plant variety with reduced allelopathy.

10. A method for breeding a plant variety with enhanced resistance to weeds, the method comprising the following step: increasing the content of isoorientin in a recipient plant to obtain an enhanced resistance to weeds.

11. A method for breeding a plant variety with reduced resistance to weeds, the method comprising the following step: reducing the content of isoorientin in a recipient plant to an reduced resistance to weeds.

12. The method according to claim 8 or 10, the method comprising increasing the content of isoorientin in the recipient plant by the following steps:
(A1) reducing the activity and/or expression of protein which is capable of catalyzing the transformation of isoorientin into other substances in the recipient plant; and/or
(A2) increasing the activity and/or expression of protein which is capable of catalyzing the transformation of other substances into isoorientin in the recipient plant.

13. The method according to claim 9 or 11, the method comprising reducing the content of isoorientin in the recipient plant by the following steps:
(B1) increasing the activity and/or expression of protein which is capable of catalyzing the transformation of isoorientin into other substances in the recipient plant; and/or
(B2) reducing the activity and/or expression of protein which is capable of catalyzing the transformation of other substances into isoorientin in the recipient plant.

14. The method according to claim 12, **characterized in that** the (A1) is implemented by (a1); and the (A2) is implemented by (a2):
(a1) inhibiting the expression of a gene encoding a protein which is capable of catalyzing the transformation of isoorientin into other substances in the recipient plant; and
(a2) introducing a gene encoding a protein which is capable of catalyzing the transformation of other substances into isoorientin in the recipient plant.

15. The method according to claim 13, **characterized in that** the (B1) is implemented by (b1); and the (B2) is implemented by (b2):
(b1) introducing a gene encoding a protein which is capable of catalyzing the transformation of isoorientin into other substances in the recipient plant; and
(b2) inhibiting the expression of a gene encoding a protein which is capable of catalyzing the transformation of other substances into isoorientin in the recipient plant.

16. The method according to any one of claims 12 to 15, **characterized in that** the other substances are substances that is not capable of improving the allelopathy of the recipient plant, or substances that improve the allelopathy of the recipient plants less than isoorientin.

17. The method according to any one of claims 12 to 15, **characterized in that** the other substances are substances that is not capable of improving the weed resistance to the recipient plants, or substances that improve the weed resistance to the recipient plants less than isoorientin.

18. The method according to claims 12-17, **characterized in that** in the (A1), the (a1), the (B1) and the (b1), the other substance is isoorientin-2"-O-glucopyranoside.

19. The method according to any one of claims 4-18, **characterized in that** the plant is rice; and the weed is *Echinochloa crus-galli.*

20. Use of Os02g0589400 protein or a relevant biomaterial thereof in any one of the following:
Q1, catalyzing the glycosylation of isoorientin;
Q2, preparing isoorientin-2"-O-glucopyranoside; and
Q3, regulating the content of isoorientin in plants;
wherein the relevant biomaterial is a nucleic acid molecule capable of expressing the Os02g0589400 protein or an expression cassette, a recombinant vector, a recombinant bacterium or a transgenic cell line containing the nucleic acid molecule;
the Os02g0589400 protein is a protein shown in any one of the following:
(D1) a protein with an amino acid sequence of SEQ ID No.1;
(D2) a protein having one or more amino acid(s) residues substituted and/or deleted and/or added in the amino acid sequence(s) shown in SEQ ID No.1 and having the same function;
(D3) a protein having a 99% or more, 95% or more, 90% or more, 85% or more or 80% or more homology with the amino acid sequence defined in any one of (D1) to (D2) and having the same function; and
(D4) a fusion protein obtained by attaching a tag to the N-terminal and/or C-terminal of the protein defined in any one of (D1) to (D3).

21. A plant variety breeded by the method of any one of claims 8 to 19.

22. A compound for inhibiting the growth of weeds, the compound is isoorientin.

23. The compound according to claim 22, **characterized in that** the weed is *Echinochloa crus-galli.*
